(19) Europäisches Patentamt
European Patent Office
Office européen des brevets

(11) Publication number : **0 537 070 A1**

# (12) EUROPEAN PATENT APPLICATION

(21) Application number : **92402740.2**

(22) Date of filing : **08.10.92**

(51) Int. Cl.[5] : **A61K 31/445,** A61K 31/495, A61K 47/32, A61K 47/44

(30) Priority : **08.10.91 JP 287220/91**

(43) Date of publication of application : **14.04.93 Bulletin 93/15**

(84) Designated Contracting States : **AT BE CH DE DK ES FR GB GR IE IT LI LU MC NL PT SE**

(71) Applicant : **TERUMO Kabushiki Kaisha**
**44-1 Hatagaya 2-chome Shibuya-ku**
**Tokyo (JP)**

(72) Inventor : **Watanabe, Eiji**
**1-7-17, Takamoridai**
**Isehara-shi, Kanagawa-ken (JP)**
Inventor : **Koyama, Sawako**
**2-2-4-506, Minamigaoka**
**Hadano-shi, Kanagawa-ken (JP)**
Inventor : **Tanaka, Tetsuo**
**4-1-5, Funabashi, Setagaya-ku**
**Tokyo (JP)**
Inventor : **Suzuki, Masahiro**
**413-1, Shibusawa**
**Hadano-shi, Kanagawa-ken (JP)**

(74) Representative : **Gillard, Marie-Louise et al**
**Cabinet Beau de Loménie 158, rue de l'Université**
**F-75340 Paris Cédex 07 (FR)**

## (54) Compositions containing compound TMK688 and its analogs.

(57) Disclosed herein is a solid formulation containing an amide derivative represented by the following formula (I), polyethylene glycol hydrogenated castor oil, and polyvinylpyrrolidone.
Formula (I)

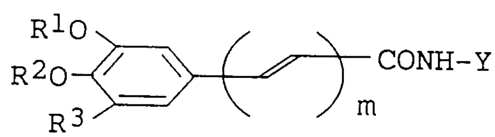

(I)

In the formula (I), $R^1$ and $R^2$ are identical or different, each being alkyl group, benzyl group, tetrahydrofuranyl group, alkoxyalkyl group or alkoxycarbonyl group, $R^3$ is hydrogen atom or alkoxy group, m is 1 or 2, and Y is a group represented by the following formula (II), (III) or (IV).
Formula (II)

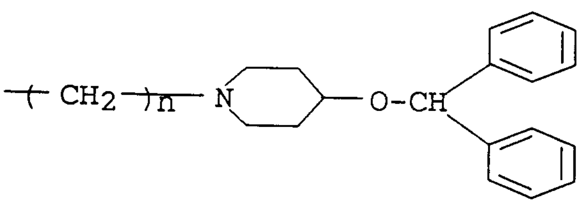

(II)

Formula (III)

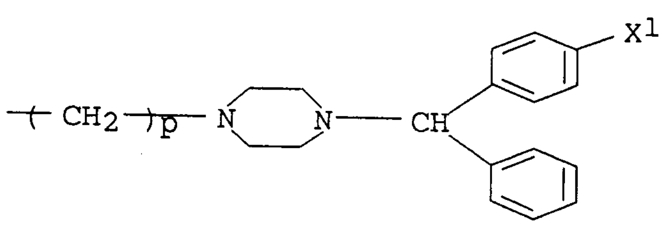

(III)

Formula (IV)

EP 0 537 070 A1

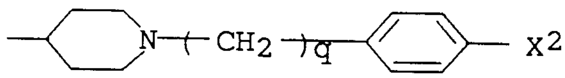

(IV)

In the formulas (II) to (IV), $X^1$ and $X^2$ are identical or different, each being a hydrogen atom or alkoxy group, and n, p and q are integers, each ranging from 1 to 4. The solid formulation can be manufactured by mixing an amide derivative represented by the formula (I), polyethylene glycol hydrogenated castor oil and polyvinylpyrrolidone with a solid carrier or an excipient in the presence or absence of a solvent.

The present invention relates to a solid formulation for an oral administration, containing a specific amide derivative, and also to a method of manufacturing the solid formulation. More particularly, it relates to a solid formulation containing an amide derivative which can be dissolved readily and be absorbed well.

The inventors have developed an amide derivative which is represented by the following formula (I) and also an anti-allergy drug which contains the amide derivative, and have filed a patent application (U.S. Patent No. 4,795,752).

Formula (I)

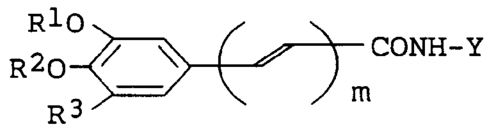

(I)

where $R^1$ and $R^2$ may be identical or different, each being alkyl group, benzyl group, tetrahydrofuranyl group, alkoxyalkyl group or alkoxycarbonyl group, $R^3$ is hydrogen atom or alkoxy group, m is 1 or 2, and Y is a group represented by the following formula (II), (III) or (IV):

Formula (II)

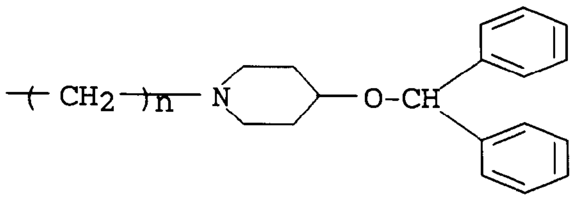

(II)

Formula (III)

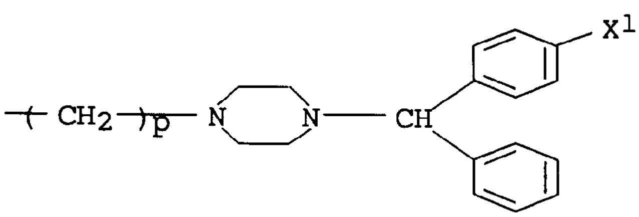

(III)

Formula (IV)

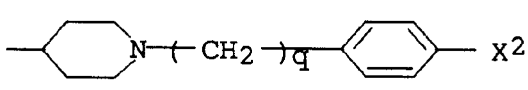

(IV)

where $X^1$ and $X^2$ may be identical or different, each being a hydrogen atom or alkoxy group, and n, p and q are integers, each ranging from 1 to 4.

The amide derivative of formula (I), which the inventors have developed, can inhibit 5-lipoxygenase and work against histamine. Also it is very effective as a drug for preventing and healing allergic diseases such as bronchial asthma, allergic rhihnitis, atopic dermatitis and pollinosis. This amide derivative can be administered in the form of a solid drug or liquid medicine.

Hitherto, to process a drug into solid formulations for oral administration, lactose, starch and a small amount of an aqueous solution of cellulose derivative are added to the drug. The mixture is kneaded and granulated by a extruder. The solid formulation made by processing the amide derivative of the formula (I) in the same way, however, was not dissolved or absorbed as much is desired. Consequently, the amide derivative could not exhibit the excellent anti-allergy property inherent in it.

A method of manufacturing solid formulations for oral administration such that the drug used may be dissolved readily is disclosed in Published Unexamined Japanese Patent Application No. 57-206612. In this method, polyvinylpyrrolidone and polyethylene glycol are added to nifedipine which is a drug for circulatory organs, thus forming an organic solution. The organic solution is sprayed on anhydrous calcium hydro genphosphate. The resultant substance is granulated, thereby forming oral solid formulations. When processed by this method, however, the amide derivative represented by the formula (I) could not be dissolved or absorbed as much as is desired.

Accordingly the object of the present invention is to provide a solid formulation which contains the amide derivative of the formula (I) as medically effective substance and which can be dissolved readily and absorbed well.

After repeatedly conducting researches and studies, the inventors of the present invention have found that the use of polyvinylpyrrolidone and polyethylene glycol hydrogenated caster oil makes it possible to manufacture a solid formulation which contains the amide derivative of the formula (I) and which can be dissolved and absorbed well, and which does not develop side effects such as diarrhea. The present invention is based on this specific finding of the inventors .

According to the invention, there is provided a solid formulation which contains an amide derivative represented by the following formula (I), polyethylene glycol hydrogenated castor oil, and polyvinylpyrrolidone:

Formula (I)

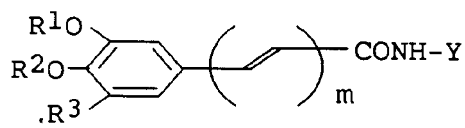

(I)

where $R^1$ and $R^2$ may be identical or different, each being alkyl group, benzyl group, tetrahydrofuranyl group, alkoxyalkyl group or alkoxycarbonyl group, $R^3$ is hydrogen atom or alkoxy group, m is 1 or 2, and Y is a group represented by the following formula (II), (III) or (IV):

Formula (II)

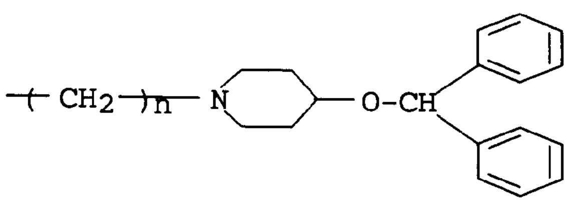

(II)

Formula (III)

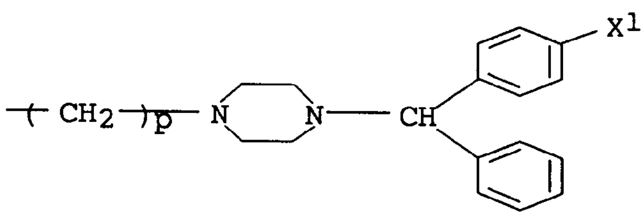

(III)

Formula (IV)

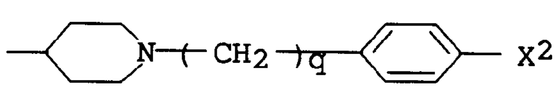

(IV)

where $X^1$ and $X^2$ may be identical or different, each being a hydrogen atom or alkoxy group, and n, p and q are integers, each ranging from 1 to 4.

According to the invention, there is provided a method of manufacturing a solid formulation which is characterized in that an amide derivative represented by the formula (I), polyethylene glycol hydrogenated castor oil, and polyvinylpyrrolidone are mixed with a solid carrier or an excipient in the or presence or absence of a solvent.

The solid formulation formed of not only an amide derivative of the formula (I) but also polyvinylpyrrolidone and polyethylene glycol hydrogenated castor oil is improved in terms of the dissolution of the amide derivative and, hence, the absorption thereof. When this solid formulation is orally administered, the amide derivative is fast dissolved and absorbed, effectively healing or preventing allergic diseases such as bronchial asthma, allergic rhinitis, atopic dermatitis and pollinosis.

As has been indicated, the amide derivative used in the invention is represented by the formula (I) and is hereinafter referred to as amide derivative (I). If, in the formula (I), $R^1$ and $R^2$ are alkyl groups, alkoxyalkyl groups or alkoxycarbonyl groups, $R^3$ is alkoxy group, and $X^1$ and $X^2$ in the group Y is alkoxy groups, the alkyl groups and the alkoxy groups are preferably those having 1 to 4 carbon atoms. If $X^1$ and $X^2$ in the group Y is halogen atoms, they are preferably chlorine, fluorine, or bromine.

The amide derivative (I) used in the invention is crystalline powder. It can be easily dissolved in an organic solvent such as acetone, dichloromethane, or trichloromethane, but can hardly be dissolved in water.

A typical example of the amide derivative (I) is 1-[{5′-(3′-methoxy-4′-ethoxycarbonyloxyphenyl)-2′,4′-pentadienoyl}aminoethyl]-4-diphenylmethoxypiperazine (hereinafter referred to as TMK 688) which is represented by the following formula (V):

Formula (V)

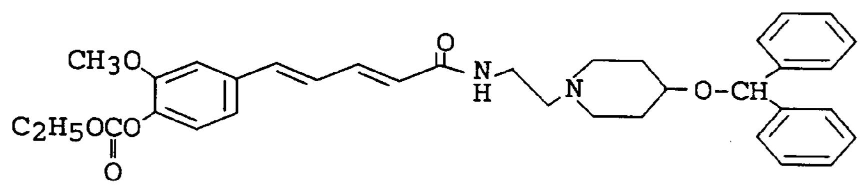

(V)

TMK 688 is pale yellow crystalline powder having a melting point of 100°C, and can easily be dissolved in dichloroethane and acetone, can be well dissolved in acetonitrile, cannot be dissolved well in ethanol, and can hardly be dissolved in water.

The amide derivative (I) for use in the present invention is not limited to the compound represented by the formula (V). Any other amide derivative that is represented by the formula (I) can be used in this invention. Use can be made of, for example, the amide derivatives identified by the following formulas (VI), (VII), and (VIII), and other various amide derivatives disclosed in U.S. Patent No. 4,795,752. These amide derivatives can be manufactured by various methods, one of which is disclosed in, for example, U.S. Patent No. 4,795,752.

Formula (VI)

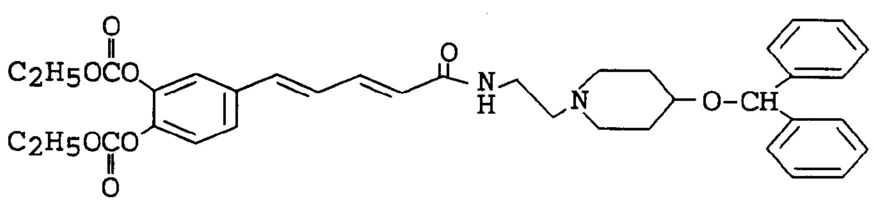

(VI)

Formula (VII)

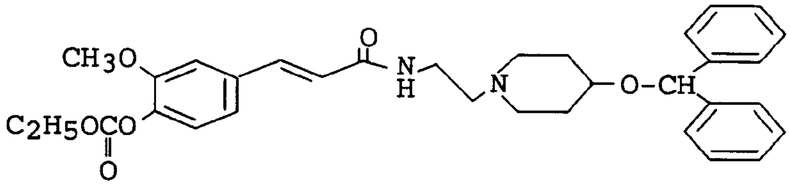

(VII)

Formula (VIII)

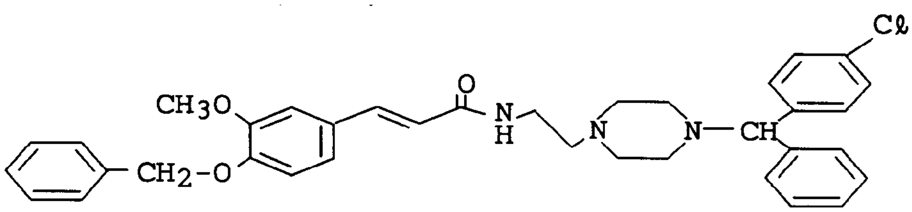

(VIII)

One of the components of the solid formulation according to this invention is polyethylene glycol hydrogenated castor oil which is a kind of a non-ionic surfactant. In the invention of Published Unexamined Japanese Patent Application No. 57-206612, polyethylene glycol, which is hydrophilic as a whole, is used as surfactant. Polyethylene glycol is not used as surfactant in the present invention. As has been described above, polyethylene glycol cannot serve to improve the dissolution or absorption of a solid formulation containing the amide derivative (I). Ionic surfactants, such as anionic surfactant, cationic surfactant and amphoteric surfactant, are not suitable for use in the solid formulation, since they are very irritant to mucous membranes and may cause diarrhea if administered orally.

Examples of the non-ionic surfactant for use in this invention are: polyethylene glycol sorbitan ester of fatty acid such as polyethyleneglycol sorbitan monooleate, polyethylene glycol monoester of a fatty acid such as polyethylene glycol monostearate, polyethylene glycol hydrogenated castor oil, sorbitan ester of fatty acid, saccharose ester of fatty acid, fatty acid monoglyceride, and copolymer of polyethylene glycol and polypropylene glycol. Of these non-ionic surfactants, polyethylene glycol hydrogenated castor oil is particularly preferable for use in the solid formulation of the present invention.

Castor oil is a triglyceride of fatty acid, the main component of the fatty acid being oleic acid having one double bond. The double bond of castor oil is saturated by adding hydrogen, whereby hydrogenated castor oil is obtained. The polyethylene glycol hydrogenated castor oil, which is preferably used in the solid formulation of this invention, is a compound prepared by introducing polyoxyethylene chains into hydrogenated castor oil, and is represented by the following formula (IX):

Formula (IX)

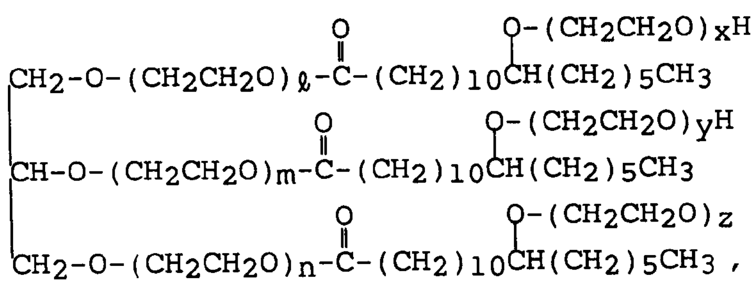

(IX)

in which the sum of l, m, n, x, y and z ranges from 20 to 100, preferably 50 to 80.

The surfactant is a component indispensable for improving the dissolution of the amide derivative. The inventors have found, that if the surfactant is used too much, the following undesirable consequences will take place:

i) Softening of feces

ii) Increase in the formulation size, making it hard for a patient to swallow the formulation

iii) Cracking of a capsule due to water absorption through the sheath.

Therefore, the inventors conducted researches for the purpose of reducing the amount of the surfactant, without losing the advantage of the present invention. They found that the purpose can be attained by using polyvinylpyrrolidone together with the surfactant. When polyvinylpyrrolidone is used along with polyethylene glycol hydrogenated castor oil functioning as a surfactant, the dissolution of the resultant solid formulation is surprisingly not impaired even if the polyethylene glycol hydrogenated castor oil is used in an amount which is only one tenth the amount used in the case where no polyvinylpyrrolidone is utilized. This makes it possible to manufacture small solid formulations according to the invention.

It is desirable that polyvinylpyrrolidone for use in the solid formulation of this invention be one which has an average molecular weight of 2,000 to 400,000, more preferably 10,000 to 200,000.

The solid formulation according to the invention can be manufactured by mixing the amide derivative (I), polyethylene glycol hydrogenated castor oil and polyvinylpyrrolidone with a solid carrier or excipient, and mechanically processing the resultant mixture into solid formulations of any desired dosage form. The formulations may be manufactured in the form of granules, subtilized granules, capsules, powder, tablets, sugar-coated tablets, pellets, and the like.

Two methods are available to mix the amide derivative (I), polyethylene glycol hydrogenated castor oil and polyvinylpyrrolidone with a solid carrier or excipient. The first method is to mix the three components, each in the form of powder, with the carrier or excipient, and to heat the resultant mixture, thereby dissolving or dispersing the amide derivative (I). The second method is to dissolve or disperse the three components, each in part or entirety, in an organic solvent or a mixture of an organic solvent and water, and to mix the resultant solution with the carrier, the excipient, or solid material containing the rest of three components.

In the second method, polyvinylpyrrolidone may be dissolved in a solvent and then be mixed with the carrier or excipient, or may be mixed, in the form of powder, with the carrier or excipient. The other components, i.e., the amide derivative (I) and polyethylene glycol hydrogenated castor oil should preferably be dissolved or dispersed in a solvent before it is mixed with the carrier or excipient.

When the solid formulations have a dosage form such as granule, subtilized granule, tablet, sugar-coated tablet, pellet, or the like, which is made using granulation or shaping, the wet mixture prepared by the second method is processed preferably by an appropriate method such as kneading extrusion, stirring granulation, rolling granulation, fluidized bed granulation, compacting granulation, or the like and is then dried, whereby the solid formulation is manufactured.

The solid carrier or excipient for use in the solid formulation of this invention may be any carrier or excipient that is commonly used in manufacturing solid formulations. Examples of the solid carrier and the solid excipient are: inorganic substances such as light anhydrous silicic acid, calcium silicate, magnesium aluminate metasilicate, calcium carbonate, calcium phosphate, and talc; starches such as potato starch and corn starch; and organic substances such as lactose, dextrin, alginic acid, mannitol, and magnesium stearate. One or more of these solid carriers or excipients can be used in this invention.

The amide derivative (I), polyethylene glycol hydrogenated castor oil, and polyvinylpyrrolidone are used in a weight ratio of, preferably, 1 : about 0.1 to 10 : about 0.1 to 5, more preferably 1 : about 0.3 to 5 : about 0.3 to 3, so that the resultant solid formulation may be dissolved and absorbed well.

It is desirable that the solid carrier or excipient be used in an amount about 1 to 200 times by weight greater than the amide derivative (I).

The solid formulation of this invention may contain, if necessary, additives such as a colorant, a flavor, a sweetener, a bulking agent, a disintegrant, and a lubricant.

The present invention will now be described in detail, with reference to some examples. Nonetheless, the invention is not limited to these examples.

Experiment 1

The mutual action of TMK 688 and each of various surfactants was examined and analyzed.

I. Preparation of Samples

One gram of TMK 688 and 5g of each of the surfactants specified in Table 1 (hereinafter presented) were heated in a water bath, stirred together, and cooled, thus preparing a sample.

II. Stability Test

The samples, thus prepared, were stored for one month at 60°C. Then, TMK 688 in each sample was quantitatively analyzed by means of high pressure liquid chromatography, thereby evaluating the stability of TMK 688 in terms of the ratio of TMK 688 remained to the initial amount thereof.

i) Preparation of Sample Solutions

A portion of each sample was poured into a test tube, in such an amount that 10 mg of TMK 688 is contained in the test tube. Then, 10 mℓ of a solution prepared by dissolving 1g of cinnamic acid in 10,000 mℓ of a buffer solution (hereinafter referred to as "solution A"), pH 3.0, formed of acetonitrile and 0.04M ammonium phosphate (55 : 45) was added, as an internal standard substance, to the sample in the test tube. Next, solution A was further added to the test tube, thereby forming 100 mℓ of a sample solution.

Solution A had been prepared in the following way. First, water was added to 2.75 mℓ of 85 w/v% phosphoric acid, thereby forming 200 mℓ of an aqueous solution. Diluted ammonia water prepared by dissolving 1 mℓ of ammonia in 3 mℓ of water was added to the aqueous solution, adjusting the pH value thereof to 3.0. Water was added to the pH-adjusted aqueous solution, forming 1000 mℓ of a solution. Next, 900 mℓ of this solution was added to 1100 mℓ of acetonitrile, thus preparing solution A.

ii) Preparation of Standard Solution

About 0.1g of TMK 688 powder was accurately measured out, and dissolved in solution A, forming a solution of exactly 100 mℓ. Exactly 10 mℓ of this solution was measured out. Meanwhile, 1g of cinnamic acid was added to 10,000 mℓ of solution A, thereby forming a solution. Ten milliliters of this solution was added to 10 mℓ of the solution prepared by dissolving TMK 688 in solution A. Further, solution A was added to the resultant solution, forming 100 mℓ of standard solution.

iii) quantitative Analysis

Each sample solution obtained in i) and the standard solution prepared in ii) were sampled out, each in an amount of 20 μℓ. TMK 688 in the sample solution and the standard solution was quantitatively analyzed by the liquid chromatography defined by Japan Pharmacopoeia Test Rules, under the conditions specified below. More precisely, the peak hight $H_T$ of the TMK 688 and the peak hight $H_S$ of the internal standard substance were measured, and the ratio of $H_T$ in respect to $H_S$ was determined. Using the ratio, thus determined, the content of TMK 688 in the sample solution was calculated, as follows:

$$\text{TMK 688 content (mg)} = H_T/H_S \times 10$$

| Analysis Conditions | |
|---|---|
| Detector: | Ultraviolet-absorbing photometer (measuring wavelength: 280 nm) |
| Column: | Stainless steel tube, inner diameter: 4.6 mm, length: 250 mm |
| Filler: | Octadecylsililated silica gel particles having a diameter of 5 μm (Inertosil ODS manufactured by Gaschro Kogyo Co., Ltd.) |
| Column Temperature: | Room temperature |
| Moving Bed: | Solution A |
| Flow Rate: | 1 mℓ/min |

The results of the stability test, thus performed, were as is shown in Table 1.

III. Appearance of Sample Solutions

One gram of each sample was dissolved in 5g of water, forming an aqueous solution. The aqueous solution, thus formed, was observed to see how it looked like. The results were as is shown, also in the following Table 1.

Table 1

| Surfactant | TMK 688 remained | Appearance of solution |
|---|---|---|
| Polyethylene glycol (20) sorbitan monooleate | 71 % | Yellow, semi-transparent |
| Polyethylene glycol (60) castor oil | 55% | Yellow, semi-transparent |
| Polyethylene glycol (60) hydrogenated castor oil | 86 % | Colorless, transparent |
| Sorbitan sesquioleate | 40 % | White, opaque |
| Polyethylene glycol (23) cetyl ether | 56 % | Precipitated |

As is evident from Table 1, polyethylene glycol (60) hydrogenated castor oil excels the other surfactants in its ability to stabilize TMK 688 in the formulation and also in its ability to make TMK 688 dissolve well in water. Hence, it is most desirable to use polyethylene glycol (60) hydrogenated caster oil as surfactant in the solid formulation according to the present invention. Here, the notation of (60) means the added mol number of the polyoxyethylene group represented by l + m + n + x + y + z in the formula (IX).

Example 1

First, 20 parts by weight of TMK 688, 20 parts by weight of polyvinylpyrrolidone (Colidon 30 manufactured by BASF, Inc.), and 10 parts by weight of polyethylene glycol (60) hydrogenated castor oil (Nikol HC0-60 manufactured by Nikko Chemicals Co., Ltd.) were dispersed and dissolved in 100 parts by weight of ethanol, forming a solution. This solution was gradually added to 50 parts by weight of lactose (defined by Japan Pharmacopoeia, manufactured by NCZ, Inc.) and 50 parts by weight of light anhydrous silicic acid (manufactured by Tomita Pharmaceutical Co., Ltd.), both contained in a mixer (PN-I manufactured by Iriye Shokai Co., Ltd.), while these components were being kneaded, thereby forming kneaded material. The material was fed into an extruding pelletizer (Pelleter EXX-I manufactured by Fuji Powdal Co., Ltd.), which made granules of the kneaded material. The granules were then processed by a power mill (P-02 S manufactured by Sanei Seisakusho) into regulated granules. The regulated granules were dried at 50°C. Magnesium stearate was added to the dried granules, and the granules were filled into No. 3 capsules. The contents of the components in one capsule was as follows:

| Contents of Components per One Capsule | |
|---|---|
| TMK 688 | 20.0 mg |
| Polyvinylpyrrolidone | 20.0 mg |
| Polyethylene glycol (60) hydrogenated castor oil | 10.0 mg |
| Lactose | 50.0 mg |
| Light anhydrous silicic acid | 50.0 mg |
| Magnesium stearate | 0.3 mg |

Comparative Example 1

TMK 688 was sieved with 80-mesh sieve, and 20 parts by weight of TMK 688 obtained by sieving, 64.1 parts by weight of corn starch and 14.7 parts by weight of lactose (defined by Japan Pharmacopoeia, manufactured by NCZ, Inc.) were put in the mixer that was used in Example 1. These components were kneaded in the mixer, while gradually adding 5% aqueous hydroxypropyl cellulose solution, thereby forming kneaded material. The material was processed in the same way as in Example 1, into pellets. The pellets were filled into No. 3 capsules. The contents of the components in one capsule was as follows:

| Contents of Components in a Capsule | |
|---|---|
| TMK 688 | 20.0 mg |
| Corn Starch | 64.1 mg |
| Lactose | 14.7 mg |
| Hydroxypropyl cellulose | 1.1 mg |
| Magnesium stearate | 0.3 mg |

Experiment 2

The capsules prepared in Example 1 and Comparative Example 1 were tested to determine how much TMK 688 dissolve from these capsules.

A device (DT-600 manufactured by Japan Spectroscope Co., Ltd.) qualified in accordance with Japan Pharmacopoeia Test Rules was employed in the dissolution test. The dissolution test solution used was Japan Pharmacopoeia disintegration test solution No. 1 (pH 1.2). More specifically, 900 mℓ of the test solution was poured into the test device and was maintained at 37 ±1°C. The test solution was continuously stirred by paddles. The test device and a flow cell attached to a spectrophotometer (manufactured by Japan Spectroscope Co., Ltd.) were connected by a tube. A pump was used, continuously circulating the test solution through the test device and the flow cell.

The dissolution test, described above, was conducted on each of the capsules prepared in Example 1 and Comparative Example 1. The light absorption of the test solution at 316 mm was measured over two hours from the start of the dissolution test. (Note that the wavelength of 316 nm is approximately equal to the maximum absorption wavelength for TMK 688.) The ratio of TMK dissolved into the test solution was calculated from each light absorption value, thus measured, in accordance with the equation set forth below:

Ratio of TMK 688 dissolved (%) = (A/B) x 100 where A is the absorption value of the test solution at 316 nm, and B is the absorption value of a solution prepared by dissolving 20 mg of TMK 688 in 900 mℓ of test solution No. 1 at 316 nm.

The results of the dissolution test, thus performed, were as is shown in the following Table 2:

Table 2

| Dissolution time | Ratio of TMK 688 dissolved (%) | |
|---|---|---|
| | Example 1 | Comp. Example 1 |
| 0 hour | 0.0 | 0.0 |
| 0.5 hours | 72.1 | 7.2 |
| 1.0 hour | 78.6 | 9.2 |
| 1.5 hours | 82.1 | 10.9 |
| 2.0 hours | 85.9 | 12.3 |

As can be obviously understood from Table 2, TMK 688 dissolved far more faster and much more efficiently from the capsules of Example 1, which contained, in addition to TMK 688, polyvinylpyrrolidone and polyethylene glycol (60) hydrogenated castor oil, than from the capsules of Comparative Example 1, which were manufactured by the conventional method.

Example 2

First, 20 parts by weight of TMK 688, 15 parts by weight of polyvinylpyrrolidone (Colidon 30 manufactured by BASF, Inc.), and 15 parts by weight of polyethylene glycol (60) hydrogenated castor oil (Nikol HC0-60 manufactured by Nikko Chemicals Co., Ltd.) were dispersed and dissolved in 70 parts by weight of ethanol, forming a solution. This solution was gradually added to 5 parts by weight of crosslinked polyvinylpyrrolidone (Colidon CL manufactured by BASF, Inc.) and 5 parts by weight of calcium silicate (manufactured by Tokuyama Soda

Co., Ltd.), both contained in a stirring pelletizer (New Speed Kneader, manufactured by Okada Seiko Co., Ltd.), while these components were being kneaded, thereby into granules. The granules were then dried at 50°C. Further, they were processed by a power mill (P-02 S manufactured by Sanei Seisakusho) into regulated granules. Magnesium stearate was added to the dried and regulated granules, and the granules were packed into No. 3 capsules. The contents of the components in one capsule was as follows:

| Contents of Components per One Capsule | |
|---|---|
| TMK 688 | 20.0 mg |
| Polyvinylpyrrolidone (Colidon 30, BASF, Inc.) | 20.0 mg |
| Polyethylene glycol (60) hydrogenated castor oil | 10.0 mg |
| Crosslinked polyvinylpyrrolidone (Colidon CL, BASF, Inc.) | 5.0 mg |
| Calcium silicate | 5.0 mg |
| Magnesium stearate | 0.2 mg |

Experiment 3

Sixteen crossbred dogs, which had not been given any food the previous night, were divided into four groups, each consisting of four. The capsule formulations of Example 2 were orally administered to the dogs of the first group, in an amount of 1 mg per kilogram of dogs' weight. The capsule formulations of Comparative Example 1 were administered to the dogs of the second group, also in an amount of 1 mg per kilogram of dogs' weight. The capsule formulations of Example 2 were orally administered to the dogs of the third group, in an amount of 20 mg per kilogram of dogs' weight. The capsule formulation of Comparative Example 1 were administered to the dogs of the fourth group, in an amount of 1 mg per kilogram of dogs' weight.

Venous blood was sampled from the brachium of every dog of any group five times, 0.5 hours, 1 hour, 2 hours, 4 hours and 6 hours after the oral administration, respectively. The total amount (ng) of the compound hydrolyzed at the 4-position, i.e., metabolite of TMK 688, and the conjugate of this compound, both contained in every milliliter of the sampled blood, was measured. The average content of the compound and the conjugate combined, measured in nanograms per milliliter of blood, was calculated of the four dogs of each group. The average content, thus obtained, was as is shown in Table 3.

Based on the data of Table 3, a graph was prepared, showing four curves each indicating how the average content of the compound and the conjugate thereof, calculated of the dogs of each group, did change with time, for six hours after the administering of the capsules. Further, the integrated value $AU_{0-6}$ (ng·hr/ml) for the area below each of these curves in the graph is shown in Table 3, too.

Table 3

| | Group 1 | Group 2 | Group 3 | Group 4 |
|---|---|---|---|---|
| Capsule formulation | Ex. 2 | Comp. 1 | Ex. 2 | Comp. 1 |
| Amount (mg/weight kg) | 1 | 1 | 20 | 20 |
| Concentration of Metabolite (ng/ml) | | | | |
| 0.5 hrs later | 9.6 | 0.1 | 210.5 | 121.8 |
| 1.0 hr later | 42.9 | 7.5 | 397.9 | 218.7 |
| 2.0 hrs later | 71.9 | 17.9 | 809.1 | 250.3 |
| 4.0 hrs later | 62.4 | 25.1 | 747.2 | 252.4 |
| 6.0 hrs later | 29.7 | 16.0 | 371.2 | 261.3 |
| AU0-6 (ng·hr/ml) | 299.3 | 98.7 | 3483.7 | 1366.5 |

As is evident from Table 3, TMK 688 was dissolved and absorbed far more faster from the capsules of Example 2, which contained, in addition to TMK 688, polyvinylpyrrolidone and polyethylene glycol (60) hydrogenated castor oil, than from the capsules of Comparative Example 1, which were manufactured by the conventional method, even in the case where the capsules were administered to living bodies.

Example 3

First, 10 parts by weight of TMK 688, 15 parts by weight of polyvinylpyrrolidone (Colidon 30 manufactured by BASF, Inc.), and 10 parts by weight of the same polyethylene glycol (60) hydrogenated castor oil as used in Example 1 were dissolved in ethanol, while heating these components. As a result, a solution was formed. In the meantime, 10 parts by weight of calcium carboxymethyl cellulose (E.C.G. manufactured by Gotoku Yakuhin Co., Ltd.), 10 parts by weight of calcium silicate, 20 parts by weight of crystalline cellulose (Avicell manufactured by Asahi Kasei Co., Ltd.), and 25 parts by weight of lactose were fed into a stirring pelletizer and were mixed in the pelletizer. Then, the solution, prepared as described above, was gradually added to the mixture contained in the stirring pelletizer, while all components were being kneaded thereby forming granules. The granules were then dried at 50°C, and processed by a power mill into regulated granules. The regulated granules were mixed with 2 parts by weight of magnesium stearate, and the resultant mixture was processed by a single punch tabletting machine (KT-1 manufactured by Okada Seiko Co., Ltd.), whereby tablets having a diameter of 8 mm were prepared.

The tablets, thus prepared, had hardness of 8 $kg/cm^2$ as measured by Monsant durometer. Their disintegration time was about 5 minutes. The contents of the components in one tablet was as follows:

| Contents of Components per One Tablet | |
|---|---|
| TMK 688 | 10.0 mg |
| Polyvinylpyrrolidone | 15.0 mg |
| Polyethylene glycol (60) hydrogenated castor oil | 10.0 mg |
| Calcium carboxymethyl cellulose | 10.0 mg |
| Calcium silicate | 10.0 mg |
| Crystalline cellulose | 20.0 mg |
| Lactose | 25.0 mg |
| Magnesium stearate | 2.0 mg |

Example 4

First, 10 parts by weight of TMK 688, 15 parts by weight of the same polyethylene glycol (60) hydrogenated castor oil as used in Example 1, and 15 parts by weight of sorbitan sesquioleate (Nikol SO-15 manufactured by Nikko Chemicals Co., Ltd.) were mixed. The mixture, thus obtained, was heated to 70°C. Next, 30 parts by weight of polyvinylpyrrolidone (Colidon 30 manufactured by BASF, Inc.) was added to the mixture and mixed therewith. Thereafter, 930 parts by weight of lactose was added, thus forming a mixture. This mixture was cooled, while being stirred. To the mixture thus cooled, 3% by weight of water was added. The resultant mixture was kneaded and then passed through a 32-mesh sieve. That portion of the mixture which was sieved out was dried at 50°C, thereby obtaining a formulation in the form of granules.

The contents of the components in the granules, per 100g thereof, was as follows:

| Contents of Components per 100g of Granules | |
|---|---|
| TMK 688 | 1.0 g |
| Polyvinylpyrrolidone | 3.0 g |
| Polyethylene glycol (60) hydrogenated castor oil | 1.5 g |
| Sorbitan sesquioleate | 1.5 g |
| Lactose | 93.0 g |

## Example 5

First, 20 parts by weight of 1-[{5′-(3′,4′-diethoxycarbonyloxyphenyl)-2′4′-pentadienoyl}aminoethyl]-4-diphenylmethoxy piperazine, i.e., a compound represented by the formula (VI), 20 parts by weight of polyvinylpyrrolidone (Colidon 30 manufactured by BASF, Inc.), and 10 parts by weight of polyethylene glycol (60) hydrogenated castor oil were dissolved in an ethanol-dichloromethane mixed solvent (mixing ratio: 2 : 1), thus forming a 7.5% solution of the compound of the formula (VI). Next, 115 parts by weight of sucrose (Nonpallel 103 manufactured by Froint Sangyo Co., Ltd.) was introduced into a roll-moving bed (Spiracoater manufactured by Okada Seiko Co., Ltd.). While the sucrose was being stirred in the roll-moving bed, the solution was sprayed onto the sucrose. The contents of the bed was dried and packed into No. 3 capsules, thus forming capsule formulations. The contents of the components in one capsule was as follows:

| Contents of Components per One Capsule | |
|---|---|
| Compound of the formula (VI) | 20.0 mg |
| Polyvinylpyrrolidone | 20.0 mg |
| Polyethylene glycol (60) hydrogenated castor oil | 10.0 mg |
| Sucrose | 115.0 mg |

## Experiment 4

The formulations prepared in Examples 3 to 5 were tested to determine how much TMK 688 or the compound of the formula (VI) dissolve from these formulations, in the same way as in Experiment 2. The results of this dissolution test were as is shown in the following Table 4. The amount (conversion value) of the amide derivative used in each test is shown in Table 4, too.

Table 4

| | Ex. 3 | Ex. 4 | Ex. 5 |
|---|---|---|---|
| Amine derivative | TMK 688 | TMK 688 | F.(VI) |
| Amount used (mg) | 10 | 10 | 20 |
| Dissolution ratio (%) | | | |
| 0 hr later | 0.0 | 0.0 | 0.0 |
| 0.5 hrs later | 62.1 | 82.3 | 75.4 |
| 1.0 hrs later | 74.8 | 88.1 | 83.4 |
| 1.5 hrs later | 82.0 | 92.6 | 88.3 |
| 2.0 hrs later | 90.2 | 98.3 | 92.1 |

As is evident from Table 4, the amide derivative (I) was dissolved very fast and very efficiently from the

formulations of Examples 3 to 6, which contained, in addition to the amide derivative, polyvinylpyrrolidone and polyethylene glycol (60) hydrogenated castor oil.

Example 6

First, 10 parts by weight of 1-[{3′-(3′-methoxy-4′-ethoxycarbonyloxyphenyl)-2′-propenoyl}aminoethyl]-4-diphenylmethoxy piperazine, i.e., a compound represented by the formula (VII), 15 parts by weight of polyethylene glycol (60) hydrogenated castor oil, and 15 parts by weight of sorbitan sesquioleate (Nikol SO-15 manufactured by Nikko Chemicals Co., Ltd.) were dissolved in 10-folds amount of ethanol. The resultant solution was gradually added to 30 parts by weight of polyvinylpyrrolidone (Colidon 30 manufactured by BASF, Inc.) and 930 parts by weight of lactose, already contained in a stirring pelletizer, while these components were being stirred, thereby forming granules. The granules were dried at 50°C, thereby obtaining a formulation in the form of granules.

The contents of the components, per 100g of the granules, was as follows:

| Contents of Components per 100 g of Granules | |
|---|---|
| Compound of the formula (VII) | 1.0 g |
| Polyvinylpyrrolidone | 3.0 g |
| Polyethylene glycol (60) hydrogenated castor oil | 1.5 g |
| Sorbitan sesquioleate | 1.5 g |
| Lactose | 93.0 g |

**Claims**

1. A solid formulation, containing an amide derivative represented by the following formula (I), polyethylene glycol hydrogenated castor oil, and polyvinylpyrrolidone:
Formula (I)

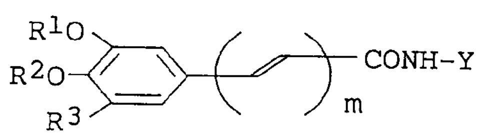

(I)

where $R^1$ and $R^2$ are identical or different, each being alkyl group, benzyl group, tetrahydrofuranyl group, alkoxyalkyl group or alkoxycarbonyl group, $R^3$ is hydrogen atom or alkoxy group, m is 1 or 2, and Y is a group represented by the following formula (II), (III) or (IV):
Formula (II)

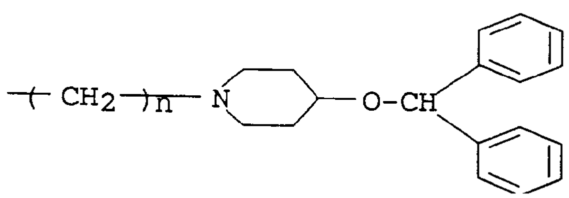

(II)

Formula (III)

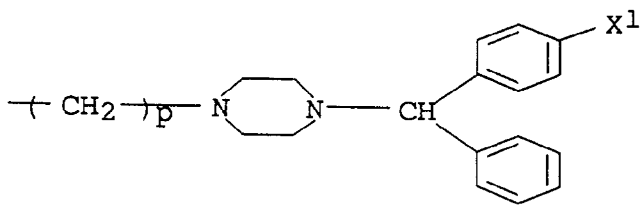

(III)

Formula (IV)

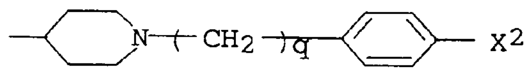

(IV)

where $X^1$ and $X^2$ are identical or different, each being a hydrogen atom or alkoxy group, and n, p and q are integers, each ranging from 1 to 4.

2. The solid formulation according to claim 1, characterized in that the amide derivative represented by the formula (I) is a compound selected from the group consisting of compounds represented by the following formulas (V), (VI), (VII) and (VIII):
Formula (V)

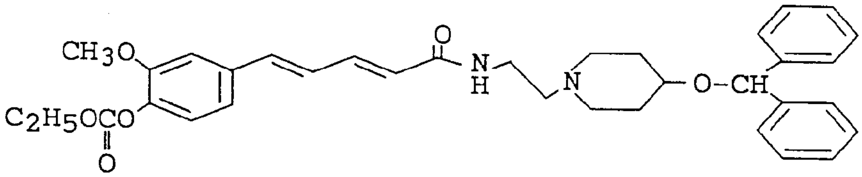

(V)

Formula (VI)

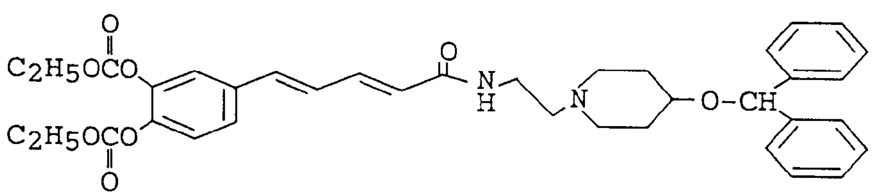

(VI)

Formula (VII)

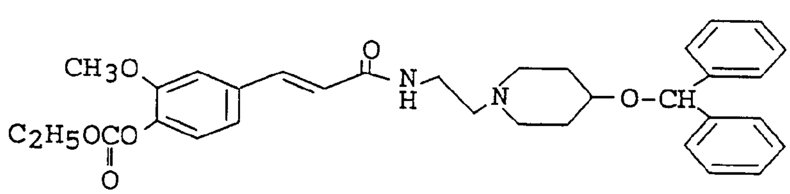

(VII)

Formula (VIII)

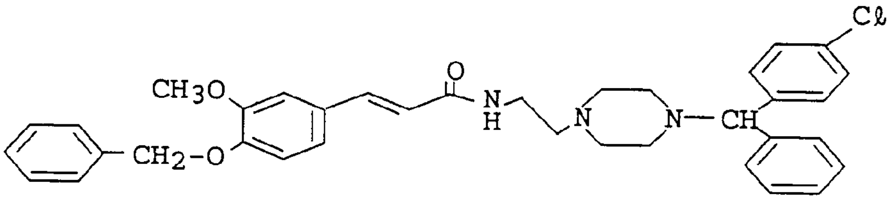

(VIII)

3. A method of manufacturing a solid formulation, characterized in that an amide derivative represented by the following formula (I), polyethylene glycol hydrogenated castor oil, and polyvinylpyrrolidone are mixed with a solid carrier or an excipient in the presence or absence of a solvent:

Formula (I)

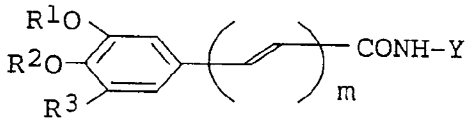

(I)

where $R^1$ and $R^2$ are identical or different, each being alkyl group, benzyl group, tetrahydrofuranyl group, alkoxyalkyl group or alkoxycarbonyl group, $R^3$ is hydrogen atom or alkoxy group, m is 1 or 2, and Y is a group represented by the following formula (II), (III) or (IV):

Formula (II)

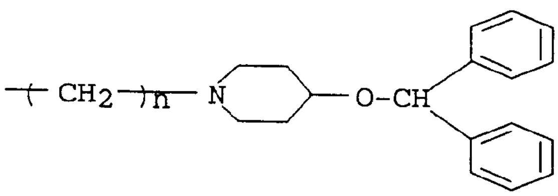

(II)

Formula (III)

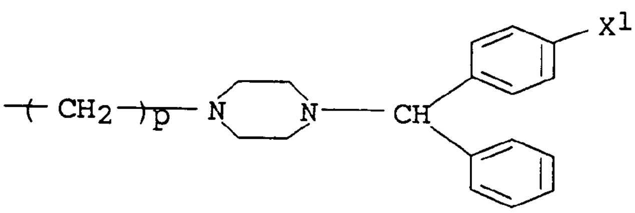

(III)

Formula (IV)

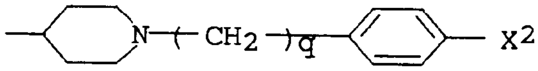

(IV)

where $X^1$ and $X^2$ are identical or different, each being a hydrogen atom or alkoxy group, and n, p and q are integers, each ranging from 1 to 4.

European Patent Office

# EUROPEAN SEARCH REPORT

Application Number

EP 92 40 2740

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 226 516 (TERUMO)<br>* page 3, line 38 - line 44; claims * | 1-3 | A61K31/445<br>A61K31/495<br>A61K47/32<br>A61K47/44 |
| A | WO-A-8 804 169 (TERUMO)<br>* the whole document * | 1-3 | |
| A | EP-A-0 157 420 (TERUMO)<br>* page 7, line 5 - line 12; claims * | 1-3 | |
| | | | TECHNICAL FIELDS SEARCHED (Int. Cl.5) |
| | | | A61K |

The present search report has been drawn up for all claims

| Place of search | Date of completion of the search | Examiner |
|---|---|---|
| THE HAGUE | 10 DECEMBER 1992 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)